# EUROPEAN PATENT SPECIFICATION

(11) **EP 0 656 418 B1**
(45) Date of publication and mention of the grant of the patent: **04.12.2002**
(21) Application number: 94119071.2
(22) Date of filing: 25.09.1984
(51) Int. Cl.: C12N 15/53, C12N 9/02

(54) **Superoxide dismutase and expression in microorganisms**
Klonieren von Superoxiddismutase und Expression in Mikroorganismen
Clonage de la dismutase du peroxyde et expression dans des microorganismes

(30) Priority: 03.10.1983 US 538607; 11.05.1984 US 609412
(43) Date of publication of application: 07.06.1995
(62) Divisional of application: 89110038.0
(73) Proprietor: CHIRON CORPORATION, Emeryville, California 94608 (US)
(72) Inventor: Hallewell,Robert A, San Francisco, California 94109 (US); Mullenbach,Guy T, California 94611 (US)
(74) Representative: Goldin, Douglas Michael

(56) References cited:
- PROCEEDINGS OF THE NATIONAL ACADEMY OF SCIENCES OF USA., vol.80, September 1983, WASHINGTON US pages 5465 - 5469 L. SHERMAN ET AL. 'Nucleotide Sequence and expression of human chromosome 21-encoded'
- BIOCHEMICAL SOCIETY SYMPOSIA, vol.48, 1983, LONDON, UK pages 233 - 244 H.A. DE BOER ET AL. 'A hybrid promoter and portable shine-dalgarno regions'

## Description

### DESCRIPTION OF SPECIFIC EMBODIMENTS

### Field of the Invention

Superoxide dismutase ("SOD") is in fact a variety of different enzymes found in most living organisms. One function in mammals is to destroy superoxide, a material naturally produced during phagocytosis. The superoxide dismutases are characterized in families based on the metal associated with the enzyme, where the metals vary amongst iron, manganese, copper and copper-zinc. Superoxide dismutase, e.g., from bovine liver, has found clinical use, particularly as an anti-inflammatory agent in mammals including humans. Other utilities include scavenging superoxide anions due to exposure of a host to various superoxide-inducing agents, e.g. radiation, paraquat, etc.; prophylaxis or therapy for certain degenerative diseases, e.g., emphysema; food preservation; and the like.

It is therefore important that stable supplies of physiologically acceptable superoxide dismutase be made available, particularly for use in vivo as an anti-inflammatory agent or for other therapeutic purposes. For human application it would be preferable to employ the homologous enzyme to prevent or minimize possible immune response. By employing recombinant DNA techniques, there is the opportunity to produce products efficiently, which have the desired biological activities of superoxide dismutase, such as immunological and enzymatic activities.

### Description of the Prior Art

The amino acid sequence of human erythrocyte Cu-Zn superoxide dismutase is described in Jabusch et al., Biochemistry (1980) 19:2310-2316 and Barra et al., FEBS Letters (1980) 120:53:55. Bovine erythrocyte Cu-Zn SOD is described by Steinman et al., J. Biol. Chem. (1974) 249:7326-7338. A SOD-1 cDNA clone is described by Lieman-Hurwitz et al., Proc.Natl.Acad.Sci.USA (1982) 79:2808-2811. Concerning the effect on efficiency of translation of varying the untranslated region upstream from the initiation codon, see Gheysen et al., Gene (1982) 17 :55-63; Thummel et al., J. Virol. (1981) 37 :683-697; and Matteucci and Heyneker, Nucl. Acids Res . (1983) 11:3113-3121. Sherman et al., Proc. Natl. Acad. Sci USA (1983) 80:5465-5469, discloses the nucleotide sequence and expression of human chromosome 21-encoded superoxide dismutase mRNA.

### SUMMARY OF THE INVENTION

Efficient production of polypeptides demonstrating the biological activity of human Cu-Zn superoxide dismutase is demonstrated by the preparation of cDNA of the major portion of the structural gene, linking to a mixture of adapters providing for varying sequences extending from the ribosomal binding site to degenerate nucleotides in the coding region, and insertion of the complete gene with its translational signals into an expression vector. Transformation of E.coli results in efficient production of a competent polypeptide demonstrating biological activity of human Cu-Zn superoxide dismutase. The gene may be further used for combining with secretory and processing signals for secretion in an appropriate host.

Accordingly, the present invention provides a DNA construct comprising a functional vector for expression in *E. coli,* said expression vector comprising in downstream order of transcription:
(1) a promoter;
(2) a ribosomal binding site;
(3) an initiation codon, wherein a DNA spacer sequence is positioned between the ribosomal binding site and initiation codon, wherein said DNA spacer sequence, said initiation codon, and the first two codons of said human cytoplasmic Cu/Zn superoxide dismutase structural gene are provided by the sequence: XnXnXnXnATGGCXACX wherein each n is independently 1, and each X is independently A, C, T, or G, and wherein said nucleotide composition of said DNA spacer is varied to provide an expression level of at least 5% w/w of total cellular protein;
(4) a human cytoplasmic Cu/Zn superoxide dismutase structural gene, or allele thereof, in reading frame with said initiation codon having a stop codon at the 3'-terminus of said gene; and
(5) a transcription terminator,
wherein said DNA construct, upon introduction into *E. coli*, expresses non-acetylated active human cytoplasmic Cu/Zn superoxide dismutase.

The invention also provides a method for preparing a polypeptide comprising non-acetylated enzymatically active human cytoplasmic Cu/Zn superoxide dismutase that differs by no more than four amino acids from naturally occurring human cytoplasmic Cu/Zn superoxide dismutase, said method comprising growing *E.coli* containing the above DNA construct in a nutrient medium, and isolating the resulting non-acetylated enzymatically active human cytoplasmic dismutase expressed by the *E.coli.*

### BRIEF DESCRIPTION OF THE DRAWINGS

Fig. 1 indicates the DNA linker sequence and a flow diagram showing its use;
Figs. 2 and 3 are flow diagrams indicating the preparation of plot5/SOD;
Fig. 4 indicates the sequence of both the coding strand of human SOD cDNA (5'^{→}3') and the resultant translation product.
Fig. 5 illustrates the sequence of the isolated human SOD gene described in the Experimental section hereinafter.
Fig. 6 is a restriction map of the isolated human SOD gene described in the Experimental section hereinafter.

### DESCRIPTION OF SPECIFIC EMBODIMENTS

Methods and compositions are provided for the efficient expression of polypeptides demonstrating the biological activities of human Cu-Zn superoxide dismutase ("hSOD"). The methods employ a DNA sequence ("hSOD gene") encoding a substantial portion of the amino acid sequence of hSOD in conjunction with a translation initiation region optimized for expression in an *E. coli* host. The hSOD gene is inserted into an appropriate vector for expression in the host, conveniently under conditions which allow for secretion, so as to harvest the SOD product from the extracellular medium.

The structural gene for hSOD or other polypeptide includes an acetylation signal sequence at the 5'-end thereof, which signal sequence causes a yeast expression host to effect actylation. The acetylation signal sequence encodes at least the first two N-terminal amino acids in the polypeptide. The first amino acid will be either alanine, glycine or serine, while the second amino acid will be a polar or aromatic amino acid, usually being threonine, serine, aspartate or phenylanine.

In order to prepare hSOD, it is necessary to have a DNA sequence which encodes for HSOD. One manner of achieving such sequence, is to clone CDNA from messenger RNA from cells which produce hSOD. Conveniently, human liver cells may be used for this purpose. After the cDNA is cloned, where the DNA coding sequence is unknown, but at least a partial amino acid sequence is known, one may then screen the cDNA with mixtures of probes having all of the possible variations of nucleotides encoding for the particular series of amino acid residues. The choice of the residues for which the sequence encodes is somewhat arbitrary, although the residues chosen will usually be selected to minimize the number of different sequences which must be synthesized.

For hSOD, conveniently a DNA sequence encoding for at least the amino acid residues 19 to 24 can be used, particularly a probe having at least about 15 bases and not more than about 20 bases, more conveniently about 17 bases. One may then restriction enzyme digest the clones which appear to hybridize with the labeled probes, fractionate the DNA fragments and repeat the hybridization, particularly by employing a second series of probes which hybridize to DNA sequences encoding for a different series of amino acid residues in hSOD. Conveniently, these amino acid residues may be 109 to 114. One or more clones may be found which are positive to both probes and these may be used as a source for cDNA encoding for at least a substantial proportion of hSOD.

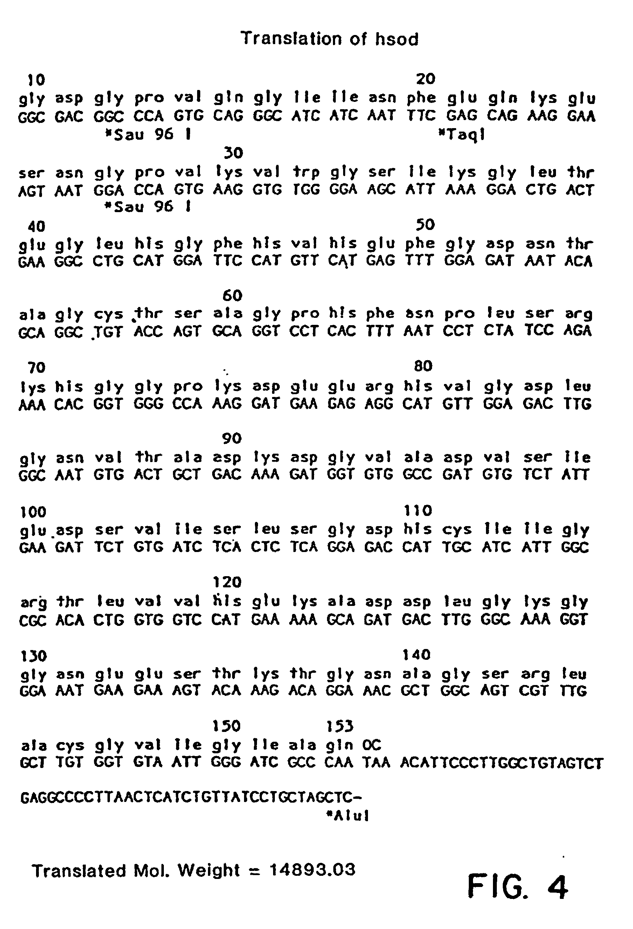

Quite surprisingly, it was found that the amino acid sequences which have been published for hSOD differed in a significant number of residues from the amino acid sequence encoded for by the cDNA. Specifically, where the two published sequences differed (Jabusch et al., Biochemistry (1980) 19:2310-2316 and Barra et al., FEBS Letters (1980) 120:153:156), the correct assignments are: residue 11, aspartate; residue 17, isoleucine; residue 26, asparagine; residue 49, glutamate; residue 52, aspartate; residue 53, asparagine; residue 92, aspartate; residue 98, serine (see Fig. 4).

Because of the uncertainties of the effect on translation of the separation between the ribosomal binding site and the translational initiation codon, normally AUG, the subject method provides a technique for varying nucleotides in the spacer between the ribosomal binding site and the initiation codon. As the base sequence downstream from the initiation site may also affect translation efficiency, the subject method also provides for variation of nucleotide sequence (but not length) within the initial 5'-codons of the polypeptide as permitted by the redundancy constraints of the genetic code. Such degeneracy may intend up to 2 codons, downstream from the initial site.

A plurality of linkers are prepared where at least 2 nucleotides, usually at least 3 nucleotides, and not more than 6 nucleotides, are varied to include members having each of the 4 nucleotides if within the spacer or 2, 3, or 4 nucleotides as permitted by genetic code redundancy if within the structural gene for the polypeptide. In addition, the linkers are prepared, having differing numbers of nucleotides, so as to provide a group of linkers differing not only in the sequence, but also in length. The difference in length can be achieved by removal of portions of the support during the linker synthesis and, if appropriate, continuing synthesis at a subsequent stage, so as to provide for linkers having a graduated number of sequence lengths. Usually, the mixture of linkers will vary in length by at least one nucleotide and not more than over a range of six nucleotides, usually not more than four nucleotides.

This can be conveniently illustrated where the absent bases are at the terminus. After each stage, a portion of the support is removed and the synthesis continued with the strands bound to the support, providing all four nucleotides (dNTP) at each stage. These single strands will then be hybridized to a single strand which is complementary in part, where the variable region will be an overhang. Thus, one will achieve a graduated series of linkers having overhangs differing in both their nucleotide sequences and lengths. At an appropriate point during subsequent hybridization, ligation or cloning operations the overhang region(s) is filled in to provide double-stranded material amenable to further manipulation. This is usually and preferably performed in vitro, e.g., using the Klenow fragment of DNA polymerase I; alternatively, in certain constructs the overhang could be cloned as a single strand with filling in occurring in vivo in the transformed or transfected host. Hybridization to a complementary strand can be achieved by having a 5'-sequence upstream from the variable nucleotide series which is complementary to a sequence present in the terminal sequence to which the linker is to be joined. The missing bases may then be filled in vitro or in vivo.

The linkers include within their sequence, at least a portion of the region between the ribosomal binding site and the initiation codon, preferably the nucleotides proximal to the initiation codon. The linker may also include the initiation codon and portions of the structural gene, the ribosomal binding site, and bases upstream from the ribosomal binding site, which may or may not include transcriptional regulatory sequences.

Usually the linker will be at least about 5 bases, more usually at least about 20 bases, and usually not exceeding about 200 bases, more usually not exceeding about 100 bases. Where the linker is greater than about 35 bases, it will usually be assembled by employing single stranded sequences of from about 10 to 35 bases, which have homology with only a part of a complementary strand, thus providing for complementary overlapping sequences with overhangs, so that the various single strands can be hybridized, ligated and the degenerate overhang filled in as indicated above to produce the desired linker having cohesive and/or blunt ends.

Where the structural gene has a convenient restriction site, usually not more than about 50 bases downstream from the initiation codon, a fragment containing the structural gene may be restricted and joined to a complementary cohesive terminus of the linker or may be filled in to provide a blunt-end terminus, which blunt end may be ligated to a blunt end of the linker. The linker is devised to ensure that the structural gene is complete and in reading frame with the initiation codon.

As indicated, in preparing the linker, one provides that there are a series of linkers which have a randomized series of nucleotides, that is, each of the four possible nucleotides in the coding strand (subject to the provision of genetic code limitations indicated above).

These linkers which are prepared from single strands may be joined to other single or double DNA strands to provide for extended linkers, which may include not only the ribosomal binding site, but bases upstream from the ribosomal binding site. Alternatively, the linkers may be relatively small, beginning at a site internal to or adjacent to the ribosomal binding site and extending downstream to a site at the initiation codon or internal to the structural gene.

While the particular order of joining the various fragments to produce the constructs of this invention will usually not be critical, conveniently, the structural gene may be first joined to the linker. This DNA construct will include not only the structural gene, but also the ribosomal binding site and any additional nucleotides upstream from the ribosomal binding site. In addition, there will be substantial variety in the nucleotides between the ribosomal binding site and initiation codon. The subject DNA construct is inserted into an appropriate expression vector which has the necessary transcriptional initiation regulatory sequences upstream, as well as transcriptional termination regulatory sequences downstream from the insertion site of the subject DNA construct. Thus, the linker will be flanked at the 5'-end with transcriptional initiation regulatory signal sequences and at the 3'-end with at least a portion of a coding region and transcriptional and translational termination sequences. (5'- and 3'-intend the direction of transcription.)

After preparing the plasmid or viral DNA for introduction into an appropriate E.coli host (usually) including at an appropriate stage in the manipulations filling in of the variable overhang region), the host is transformed or transfected, respectively, cloned, the clones streaked and individual clones selected for efficient expression by assaying for production of the desired product, e.g., hSOD. The number of clones to be screened to determine the various levels of production of the product will depend upon and be proportional to the degreee of sequence degeneracy introduced into the synthetic linker. As exemplified in the present embodiment, with 4 length variables and 4-fold sequence degeneracy at each of 6 nucleotides in the linker, the number of possible recombinant sequences is 5440. Usually at least a few hundred, preferably several thousand or more, clones will be screened. Screening can be efficiently performed using Western blots (antibody detection of product) of host cell colonies or viral plaques transferred to filters of nitrocellulase or other suitable material. Alternatively, using electrophoresis and providing for a plurality of lanes, where each lane is an individual clone, an immediate and direct comparison can be made of which clones are most efficient in expression by visualization of staining intensity, autoradiography or Western blotting of the product band. This screen will usually be sufficient, although more quantitative immunoassays or enzyme assays can be employed, as appropriate.

If desired, the construct can be transferred to a different E.coli host which recognizes the regulatory signals of the expression construct or the expression construct modified by introduction at appropriate sites of necessary regulatory signals to provide for efficient expression in an alternative host.

If desired, the hSOD gene may be joined to secretory leader and processing signals to provide for secretion and processing of the hSOD. Various secretory leader and processing signals have been described in the literature. See for example, U.S. Patent Nos. 4,336,336 and 4,338,397, as well as copending application Serial Nos. 522,909, filed August 12, 1983 and 488,857, filed April 26, 1983, the relevant portions of which are incorporated herein by reference.

Of particular interest as hosts are E. coli.

A wide variety of vectors are available for use in E.coli, the vectors being derived from plasmids and viruses. The vectors may be single copy or low or high multicopy vectors. Vectors may serve for cloning and/or expression. In view of the ample literature concerning vectors, commercial availability of many vectors, and even manuals describing vectors and their restriction maps and characteristics, no extensive discussion is required here. As is well-known, the vectors normally involve markers allowing for selection, which markers may provide for cytotoxic agent resistance, prototrophy or immunity. Frequently, a plurality of markers are present, which provide for different characteristics.

In addition to the markers, vectors will have a replication system and in the case of expression vectors, will usually include both the initiation and termination transcriptional regulatory signals, such as promoters, which may be single or multiple tandem promoters, a TATA box, enhancers, terminator, and one or more stop codons associated with the terminator. For translation, there will frequently be a ribosomal binding site as well as one or more stop codons, although usually stop codons will be associated with a structural gene. Alternatively, these regulatory sequences may be present on a fragment containing the structural gene, which is inserted into the vector.

Usually, there will be one or more restriction sites conveniently located for insertion of the structural gene into the expression vector. Once inserted, the expression vector containing the structural gene may be introduced into an E.coli host and the host cloned providing for efficient expression of hSOD.

In some instances, specialized properties may be provided for the vector, such as temperature sensitivity of expression, operators or activators for regulation of transcription, and the like. Of particular interest is the ability to control transcription by exogenous means, such as temperature, inducers, corepressors, etc., where transcription can be induced or repressed by an exogenous compound, usually organic.

Where the hSOD is made intracellularly, when the cell culture has reached a high density, the cells may be isolated, conveniently by centrifugation, lysed and the hSOD isolated by various techniques, such as extraction, affinity chromatography, electrophoresis, dialysis, or combinations thereof. Where the product is secreted, similar techniques may be employed with the nutrient medium, but the desired product will be a substantially higher proportion of total protein in the nutrient medium than in the cell lysate.

The hSOD which is formed has substantially the same amino acid sequence as the naturally occurring human superoxide dismutase, usually differing by fewer than 5 amino acids, more usually differing by fewer than 2 amino acids. The recombinant hSOD ("r-hSOD") displays substantially the same biological properties as naturally occurring hSOD. The biological properties include immunological properties, where antibodies raised to authentic hSOD cross-react with r-hSOD. Furthermore, in common bioassays employed for hSOD, the r-hSOD product demonstrates a substantial proportion, usually at least about 10%, preferably at least about 50%, more preferably at least about 80%, of the enzymatic activity of the authentic hSOD, based on weight of protein. An illustrative assay technique is described by Marklund and Marklund, Eur. J. Biochem. (1974) 47:469-474.

The following examples are offered by way of illustration and not by way of limitation.

### EXPERIMENTAL

### Molecular Cloning of hSOD cDNA

Total RNA was prepared from an adult human liver by the guanidinium thiocyanate/lithium chloride method (Cathala et al., DNA (1983) 2:329-335). polyA RNA was used to synthesize double-stranded cDNA (Maniatis et al., Molecular Cloning, 213-242, Cold Spring Harbor, 1982) and this was passed over a Sepharose CL4B column to enrich for cDNAs of greater than 350bp (Fiddes and Goodman, Nature (1979) 281:351-356). The cDNA was inserted at the PstI site of plot4, a pBR322 derivative having the following sequence replacing the PstI-EcoRI site. 
PstI    HinfI    AluI
1    GGTGAATCCGTAATCATGGTCATAGCTGTTTCCTGTGTGAAATTGTTATCCGCTC
ACGTCCACTTAGGCATTAGTACCAGTATCGACAAAGGACACACTTTAACAATAGGCGAG
HphI

HindIIAluI
60    ACAATTCCACACATTATACGAGCCGATGATTAATTGTCAACAGCTCATTTCAGAATATTT
TGTTAAGGTGTGTAATATGCTCGGCTACTAATTAACAGTTGTCGAGTAAAGTCTTATAAA

EcoRI
120    GCCAGAACCGTTATGATGCGG
CGGTCTTGGCAATACTACGCCTTAA
 The cDNA insertion employed the oligo-dG:dC tailing method (Maniatis et al., supra). E. coli strain D1210 was transformed with this mixture and transformants selected on L-agar containing 10µg/ml tetracycline (Kushner, S.R. (1978) In: Genetic Engineering, eds. Boyer, H.B. and Nicosia, S., (Elsevier/North Holland, Amsterdam) p. 17). Plasmid DNA constituting a liver cDNA library was prepared (Maniatis et al., Molecular Cloning, pp. 86-94, Cold Spring Harbor 1982) directly from approximately 62,000 recombinant colonies plated at a density of approximately 3,000 colonies per 9cm diameter Petri dish.

### Isolation of r-hSOD Clones

Strain D1210 was retransformed with the liver cDNA library and about 40,000 clones were grown on nine 14cm diameter Petri dishes. After transfer of the colonies to nitrocellulose paper and chloramphenicol amplification of plasmid DNA, the cells were lysed and the filters prepared for hybridization (Ish-Horowicz and Burke, Nucleic Acids Research (1981) 9:2989-2998). Oligonucleotide probes were employed for screening by hybridization, with the probes consisting of enzymatically-radiolabeled, chemically-synthesized DNA molecules complementary to the mRNA encoding amino acid residues 19 to 24 of the protein (Jabusch et al., supra.; Barra et al., supra.); the mixture had the following sequences: 
3' TTA AAA CTT GTT TTT CT 5'
     G  G   C   C   C
 where all of the indicated possibilities for encoding the peptide sequence were prepared (32-fold degenerate).

The probes were labeled with ³²P to a specific activity of 1-3x10⁸cpm/µg and Millipore (0.45µm) filtered before use. Filters were prehybridized for 6hrs at 30°C in 4x SSC, 2x Denhardts's solution, 40mM sodium phosphate, pH 7.5, 300µg/ml sonicated salmon testes DNA. Hybridization was for 20hrs at 30°C in the same solution containing 2x10⁶cpm/ml hSOD DNA probe (residues 19-24). Filters were washed in 4x SSC, once for 15min at r.t. and twice for 15min at 30°C, blotted dry and autoradiographed with an intensifying screen for 24hrs at -70°C.

Areas on the master plates that corresponded to duplicate positive signals were picked into L-broth and plasmid DNA prepared by the miniscreen procedure (Maniatis et al., Molecular Cloning, 178, 368-369, Cold Spring Harbor 1982). This DNA was cut with PstI and subjected to Southern blot analysis (Southern, J. Mol. Biol. (1975) 98:503-517) hybridizing initially with the previous labeled probes (amino acid residues 19-24) and then with additional radiolabeled probes derived from amino acid residues 109-114 and having the following sequences (all possible variations, 72-fold degenerate) present as a mixture: 
3' CTA GTA ACA TAA TAA CC 5'
G G G G G
T T
 One plasmid pool (pSOD1) contained a cDNA insert of 520bp that hybridized with both probes and after colony purification, plasmid DNA was prepared from this clone and sequenced by the method of Maxam and Gilbert (Proc. Natl. Acad. Sci. USA (1977) 74:560-564) with the results shown in Fig. 4. The hSOD cDNA clone pSOD1 constitutes the coding region for amino acids 10-153 of hSOD, a single translational stop codon and a 3' untranslated region. Therefore, in the expression vector construct, the base sequence of the region encoding amino acids 1-9 is derived from the published amino acid sequence of hSOD (Jabusch et al., supra; Barra et al., supra) and synthesized chemically as a part of the variable linker segment (see below).

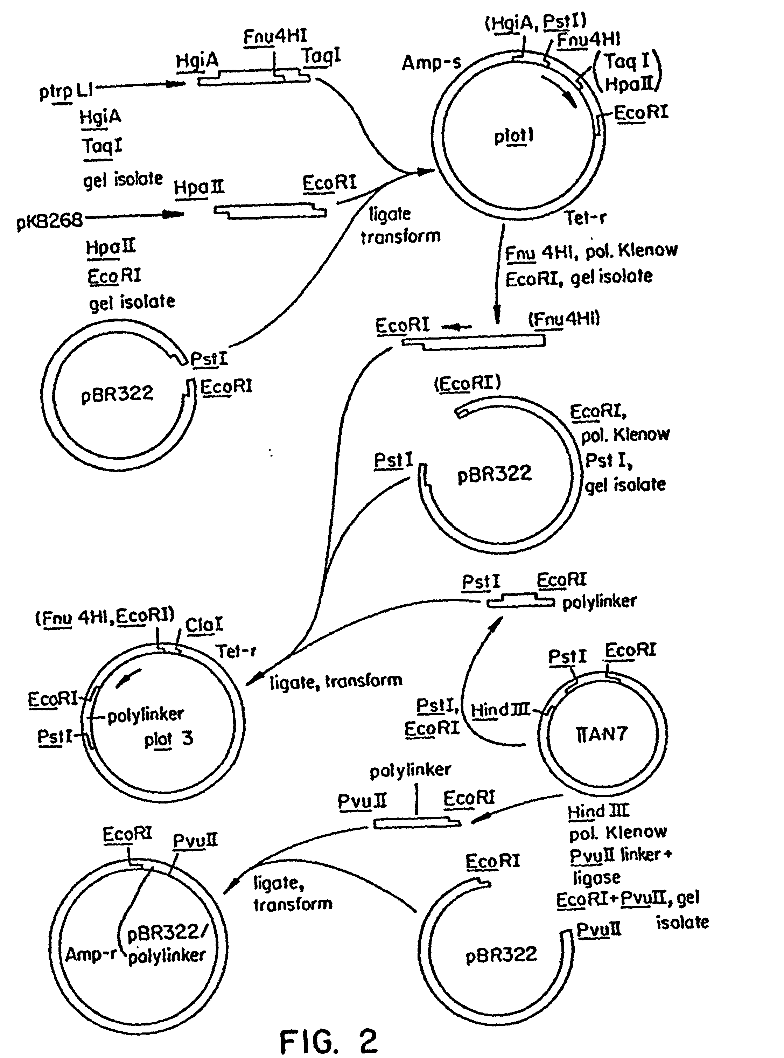

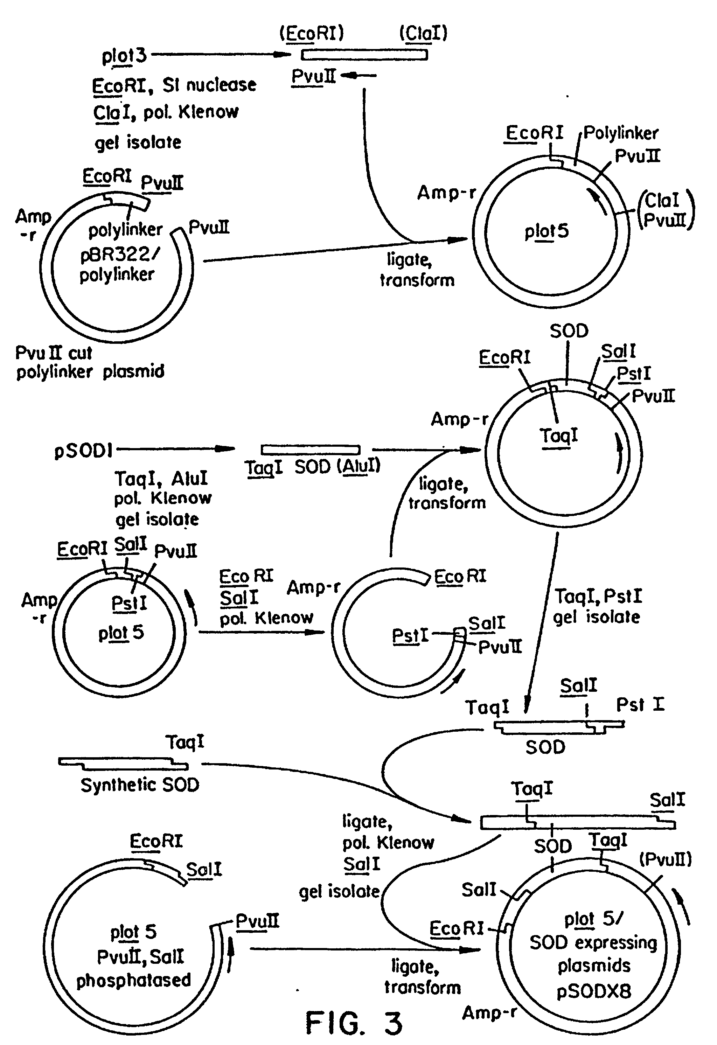

### Construction of Plasmid plot5 - (See Figures 2 and 3)

Plasmid plot1, containing a hybrid trp-lac ("tac") promoter (DeBoer et al., Proc. Natl. Acad. Sci. USA (1983) 80:21-25) was constructed by gel isolating the 180bp HgiA-TaqI fragment of ptrpL1 (Edman et al., Nature (1981) 291:503-506) and the 58bp HpaII-EcoRI fragment from pKB268 (Backman and Ptashne, Cell (1978) 13:65-71), and ligating these fragments to pBR322 digested with PstI and EcoRI. The resulting plasmid was used to transform strain D1210 and clones selected for tetracycline resistance. Plasmid plot3 was constructed by gel isolating the 155bp Fnu4HI-EcoRI fragment of plotl containing the tac promoter, with the Fnu4HI site being made flush-ended using the Klenow fragment of DNA polymerase I ("pol I K" or "pol. Klen."), and the 18bp EcoRI-PstI polylinker fragment of IIAN7 of the following sequence: 
EcoRI HindIII
5' AATTCCCGGGGATCCGTCGACCTGCAGATCTCTAGA
3' GGGCCCCTAGGCAGCTGGACGTCTAGAGATCTTCGA
SalI PstI
 These fragments were ligated to gel purified pBR322 digested with EcoRI, flush-ended using pol I K, followed by digestion with PstI and gel purified. This ligation mix was used to transform strain D1210, selecting on L-agar plates containing 10µg/ml tetracycline.

Plasmid plot5 was made by first constructing a plasmid containing the IIAN7 polylinker as an EcoRI-PvuII substitution in pBR322. To do this, plasmid IIAN7 was digested with HindIII, made flush-ended by filling in with pol I K and a synthetic, self-complementary, PvuII linker molecule (d(5'-CCAGCTGG-3')) ligated to the above-modified plasmid IIAN7. After digestion with EcoRI and PvuII, the resultant 44bp polylinker fragment (with 4-base overhangs) was gel isolated and cloned into pBR322 as an EcoRI-PvuII substitution.

Plasmid plot3 was digested with EcoRI and after phenol-chloroform extraction and ethanol precipitation, the protruding 5'-ends were made flush-ended by treatment with S1 nuclease (Palmiter, Biochemistry (1974) 13:3606-3615; Hallewell and Emtage, Gene (1980) 9:27-47). After phenol-chloroform extraction and ethanol precipitation, the DNA was digested with ClaI, made flush-ended by pol I K, and the 237bp fragment containing the tac promoter isolated by preparative polyacrylamide gel electrophoresis. This flush-ended tac promoter fragment was then inserted at the PvuII site of the pBR322 polylinker plasmid (see Fig. 3) and clones obtained in which the tac promoter directed transcription towards the β-lactamase gene of pBR322.

### Construction of plot5 Derivatives Expressing r-hSOD

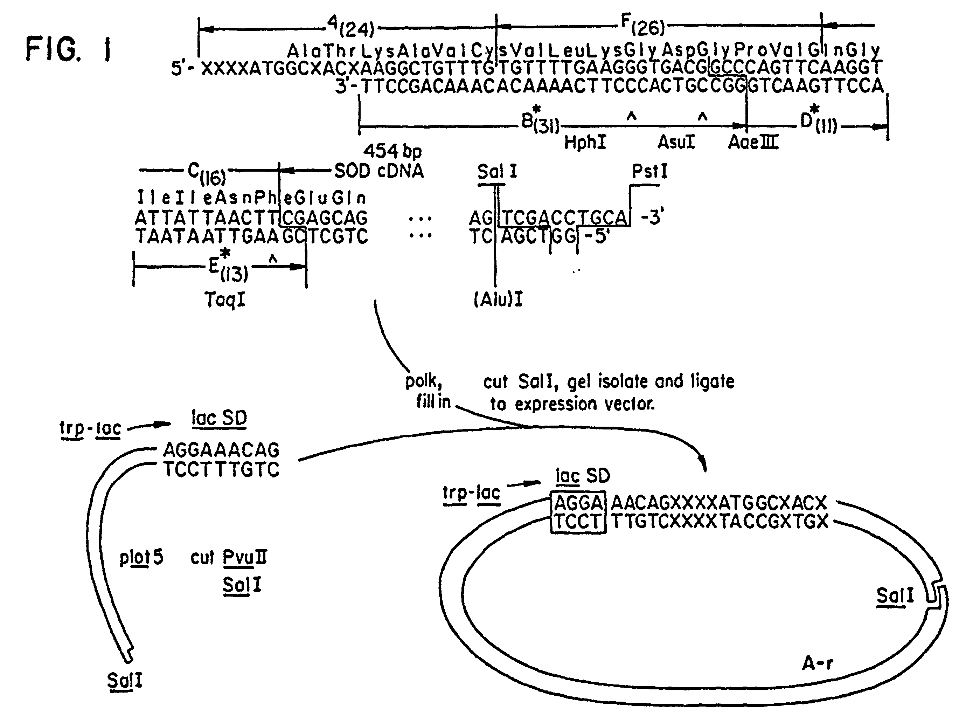

The synthetic DNA molecules F(26), C(16), B(31), D(11), E(13) and 4(24) shown in Fig. 1, were synthesized by the phosphoramidite method. The single strand 4(24) was prepared by using all four bases, at each site where X is indicated. Furthermore, silica was withdrawn from the synthesis of the 24mer, such that single-stranded 21mers, 22mers, and 23mers are obtained in addition to the 24mers. After removal from the silica support, the four mixtures are combined in appropriate proportions to provide for equimolar amounts of each of the possible single strands. This mixture was treated as a single product in the subsequent steps.

Molecules F(26), C(16), B(31) and D(11) were mixed together in equimolar amounts and 10µg phosphorylated using T4 polynucleotide kinase. After phenol-ether extraction, the additional non-phosphorylated synthetic DNA molecules 4(24) and E(13) were added, such that all fragments were equimolar. The equimolar mixture contained 13µg of DNA in 133µl of 0.3x kinase buffer.

After annealing by cooling at a uniform rate from 70°C to 20°C over 60min, the single strands were ligated together with T4 ligase in 200µl ligation mix at 14°C for 4hrs, phenol-chloroform extracted, ethanol precipitated and the 5'-ends of 4(24) and E(13) phosphorylated using T4 polynucleotide kinase (Maniatis et al., supra). Preparative polyacrylamide gel electrophoresis was used to isolate the completely ligated 53bp material having 5'- and 3'-overhangs.

The above purified fragment mixture was then ligated to the 460bp TaqI-PstI segment of the hSOD cDNA as shown in Fig. 1. This segment was itself constructed by isolating the 454bp TaqI-AluI hSOD fragment, making it flush-ended pol I K using and inserting it into plot5 between its EcoRI and Sall sites (see Fig. 3) which had been similarly made flush-ended. After preparation of plasmid DNA from this recombinant, the 460bp TaqI-PstI hSOD fragment was isolated by preparative polyacrylamide gel electrophoresis. After extraction and precipitation, the 515bp fragment resulting from the joining of the synthetic fragment to the 460bp TaqI-PstI hSOD fragment was filled in with pol I K (525-528bp) and then digested with SalI and the resulting 519-522bp hSOD fragment isolated by polyacrylamide gel electrophoresis. This fragment was then inserted into plot5 which had been digested with PvuII and SalI and then treated with alkaline phosphatase. The resulting plasmids were used to transform strain D1210. Recombinants obtained after transformation of strain D1210 were selected on L-agar containing 100µg/ml ampicillin to give a set of clones (designated plot5/SOD) with variable SOD expression.

### r-hSOD Expression and plot5/SOD Plasmid Selection

For analysis of total E. coli proteins by SDS-polyacrylamide gel electrophoresis, overnight cultures were diluted 30-fold into lml of L-broth and grown shaking at 37°C for 90min. to an O.D.₆₅₀ of about 0.2. IPTG (isopropylthiogalactoside) was added to a final concentration of 2mM and the cultures incubated an additional 3hrs. After centrifugation, the cell pellet was resuspended in 50µl of gel loading buffer (Laemmli, Nature (1970) 227:680-685) and lysed by repeating the following procedure 3x: Freezing for 1min., boiling for 2min., vortexing for 10sec.

After electrophoresis resolution (Laemmli, supra) the protein bands were stained with Coomasie blue and the amount of SOD produced by each clone estimated; these results were then confirmed using Western blots with antibody to authentic human SOD. Over three hundred clones were analyzed and exhibited levels of SOD expression varying from little or none to amounts estimated to be 5-10% of the total soluble cellular protein. Results for six of the over three hundred clones are presented in Table 1, along with the particular sequence for DNA molecule 4(24) as determined by the method of Maxam and Gilbert, supra.

**Table 1**

| Sequence and Levels of SOD Production in E . coli | | | | |
|---|---|---|---|---|
| Clone | Sequence: | | | Approximate Weight Percent of Total Protein |
| | 5'-XXXX ATG | GCX | ACX | |
| pSODX8 | AACA | A | G | 5% |
| pSOD11 | GTAT | T | G | 10% |
| pSODx16 | ACAT | A | A | 5% |
| pSOD5 | AATC | A | A | 10% |
| pSOD16 | ATTT | T | T | 10% |
| pSOD18 | AACT | A | G | 7.5% |

### Assays for r-hSOD

For enzyme assays, 100ml cultures were induced as described above. Soluble cell extracts were prepared by vortexing cell pellets 10x20sec. with an equal volume of 0.45-0.5mm diameter glass beads (Braun AG) and an equal volume of assay buffer (50mM Tris-cacodylate, pH 8.2, 1mM diethylenetriaminepentaacetic acid) in a 1.5ml microfuge tube at 4°C. Cell debris was pelleted by spinning the tube for 1min. in a microfuge (Beckman). The supernatant was dialysed against the assay buffer containing 1mM each of zinc sulfate and copper sulfate for 4hrs at 4°C.

SOD assays were performed using the pyrogallol method (Marklund and Marklund, supra). The reaction mixtures employed 0.2mM pyrogallol in assay buffer and reaction rates were determined over a 5min. period using a Hewlett-Packard 8450 spectrophotometer at 420nm. Four different assay samples were prepared: soluble E. coli extracts; authentic hSOD; and each of the prior samples pre-incubated with rabbit antibody to authentic hSOD. Each sample was incubated in a cuvette for 1min. at 25°C before adding the pyrogallol and assaying at 25°C. The antibody samples involved a preincubation of 10min. at room temperature in assay buffer with 5µl of antibody. These conditions were found to be sufficient to inactivate 100ng of pure hSOD.

The following Table 2 indicates the results for one of the clones examined (pSODx8):

**Table 2**

| Enzymatic Activity of Human Cu-Zn SOD Produced in E. Coli (strain D1210 (pSODX8)) | |
|---|---|
| Enzyme Preparation | Units SOD/mg Protein |
| pure Human Cu-Zn SOD | 15,384 |
| pSODX8 protein extract | 3,017 |
| pSODX8 protein extract preincubated with rabbit anti-human SOD antibody | 685 |
| plot5 protein extract | 470 |
| plot5 protein extract preincubated with rabbit anti-human SOD antibody | 485 |

These data indicate that approximately 15% of the total soluble cellular protein was hSOD (assuming that the pure human Cu-Zn SOD used as a reference was fully active). Taken together with the electrophoretic data (see above) indicating that 5-10% of total soluble cellular protein migrated as hSOD, it appears that a substantial fraction, probably a majority of the hSOD produced is active.

The correct sequence of the cloned gene was determined by the method of Maxam and Gilbert, supra. In addition, the first twelve amino acids at the N-terminus were determined by automated Edman degradation. The detected sequence of amino acids was as follows: ALA-THR-LYS-ALA-VAL-(CYS)-VAL-LEU-LYS-GLY-ASP-GLY- The first ALA residue detected was present at a molar concentration approximately equal to that of the input peptide indicating the absence of a blocked amino terminus. The CYS residue was not detected by the method of amino acid analysis used, but its presence was inferred from the nucleotide sequence.

Thus, the (N-formyl-) methionine was removed from the bacterial expression product and- the material had the correct amino acid sequences, i.e, identical to that reported for cytoplasmic hSOD residues 1-10, but the N-terminal ALA residue was not acetylated. Furthermore, the polypeptide made in E. coli migrated more slowly than the natural protein in 1% agarose gel (pH 8.6) electrophoresis which detects differences in charge (Corning Universal electrophoresis film, stained according to Clausen, Immunochemicar Technique, p. 530-531), also indicating lack of acetylation. In addition, analysis of tryptic peptides of both the bacterial hSOD polypeptide and the purified, authentic (acetylated) natural protein revealed that all tryptic peptides were identical, except the bacterial N-terminal peptide which migrated differently, i.e., with a charge expected for a peptide lacking the N-acetyl group.

### Isolation of the Human SOD Gene

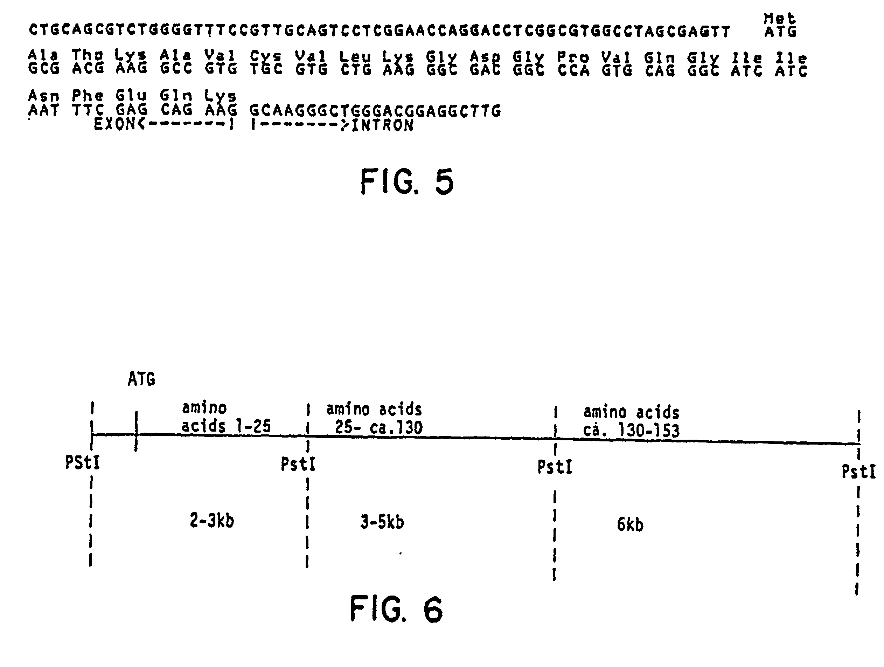

To isolate the human SOD gene, a bacteriophage lambda library representative of the human genome (R. Lawn et al. (1978) Cell 15:1157-1174) was screened with a radiolabelled DNA probe made from the human SOD cDNA. One million phage plaques were screened, and 13 positively hybridizing plaques were purified. Restriction endonuclease analysis of the phage DNAs indicated that there are at least 5 different genes, suggesting that there are other SOD related genes and gene products. One candidate for such a gene is the recently discovered extracellular Cu/Zn SOD (S. Marklund, (1982) Proc. Natl. Acad. Sci. USA 79:7634-7638). To distinguish the authentic cytoplasmic Cu/Zn SOD gene from the related ones we used synthetic DNA probes 5'-AATGCTTCCCCACACC-3' and 5'-CTCAGTTAAAATGTCTGTTTG-3' corresponding to amino acid residues 19-26 and nucleotides 193-213 3' from the terminator codon in the 3' untranslated region, respectively. Only one of the 13 genomic DNAs hybridized with these probes, indicating that it is the authentic human cytoplasmic SOD gene. This was confirmed by DNA sequence analysis of the N-terminal region, as shown in Fig. 5 where the amino acid sequence determined by protein sequencing is confirmed. This also shows that no preprotein exists for SOD since an in-frame termination codon exists nine nucleotides 5' from the initiator methionine codon. As shown in Figure 5, the human Cu/Zn SOD gene contains intervening sequences. The map of the SOD gene shown in Figure 6 indicates that there is more than one intervening sequence.

Although the foregoing invention has been described in some detail by way of illustration and example for purposes of clarity of understanding, it will be obvious that certain changes and modifications may be practiced within the scope of the appended claims.

E. coli strain D1210 (pSODX8) was deposited at the A.T.C.C. on September 27, 1983 and given Accession No. 39453. Yeast strain 2150-2-3 (pC1/1GAPSOD) and E. coli strains D1210 (pSOD11) and D1210 (pS2OR) were deposited at the A.T.C.C. on May 9, 1984, and given Accession Nos. 20708, 39679 and 39,680, respectively.

## Claims

1. A DNA construct comprising a functional vector for expression in *E. coli,* said expression vector comprising in downstream order of transcription:
(1) a promoter;
(2) a ribosomal binding site;
(3) an initiation codon, wherein a DNA spacer sequence is positioned between the ribosomal binding site and initiation codon, wherein said DNA spacer sequence, said initiation codon, and the first two codons of said human cytoplasmic Cu/Zn superoxide dismutase structural gene are provided by the sequence: XnXnXnXnATGGCXACX wherein each n is independently 1, and each X is independently A, C, T, or G, and wherein said nucleotide composition of said DNA spacer is varied to provide an expression level of at least 5% w/w of total cellular protein;
(4) a human cytoplasmic Cu/Zn superoxide dismutase structural gene, or allele thereof, in reading frame with said initiation codon having a stop codon at the 3'-terminus of said gene; and
(5) a transcription terminator,
wherein said DNA construct, upon introduction into *E. coli,* expresses non-acetylated active human cytoplasmic Cu/Zn superoxide dismutase.

2. The DNA construct according to claim 1, wherein said nucleotide composition of said DNA spacer sequence is 5'-AACAATGGCAACG-3'.

3. The DNA construct according to claim 1, wherein said nucleotide composition of said DNA spacer sequence is 5'-ACATATGGCAACA-3'.

4. The DNA construct according to claim 1, wherein said nucleotide composition of said DNA spacer sequence is 5'-AACTATGGCAACG-3'.

5. The DNA construct according to claim 1, wherein said nucleotide composition of said DNA spacer sequence is 5'-GTATATGGCTACG-3'.

6. The DNA construct according to claim I, wherein said nucleotide composition of said DNA spacer sequence is 5'-AATCATGGCAACA-3'.

7. The DNA construct according to claim 1, wherein said nucleotide composition of said DNA spacer sequence is 5'-ATTTATGGCTACT-3'.

8. The DNA construct according to claim 1, wherein said DNA construct is joined to a replication system for extrachomosomal replication and maintenance.

9. A method for preparing a polypeptide comprising non-acetylated enzymatically active human cytoplasmic Cu/Zn superoxide dismutase that differs by no more than four amino acids from naturally-occurring human cytoplasmic Cu/Zn superoxide dismutase, said method comprising:
growing *E. coli* containing a DNA construct comprising a functional vector for expression in *E. coli* in a nutrient medium, wherein said DNA construct comprises in downstream order of transcription:
(1) a promoter;
(2) a ribosomal binding site;
(3) an initiation codon, wherein a DNA spacer sequence is positioned between the ribosomal binding site and initiation codon, wherein said DNA spacer sequence, said initiation codon, and the first two codons of said human cytoplasmic Cu/Zn superoxide dismutase structural gene are provided by the sequence: XnXnXnXnATGGCXACX wherein each n is independently 1, and each X is independently A, C, T, or G, and wherein said nucleotide composition of said DNA spacer is varied to provide an expression level of at least 5% w/w of total cellular protein;
(4) a human cytoplasmic Cu/Zn superoxide dismutase structural gene, or allele thereof, in reading frame with said initiation codon having a stop codon at the 3'-terminus of said gene; and
(5) a transcription terminator,
wherein said DNA construct, upon expression, expresses non-acetylated active human cytoplasmic Cu/Zn superoxide dismutase; and
isolating the resulting non-acetylated enzymatically active human cytoplasmic superoxide dismutase expressed by said *E. coli.*

## Patentansprüche

1. Ein DNA-Konstrukt, umfassend einen funktionellen Vektor zur Expression in *E. coli,* wobei dieser Expressionsvektor in abwärtslaufender Reihenfolge der Transkription umfasst:
(1) einen Promotor;
(2) eine ribosomale Bindungsstelle;
(3) ein Startcodon, wobei eine DNA-Spacersequenz zwischen der ribosomalen Bindungsstelle und dem Startcodon angeordnet ist, wobei die DNA-Spacersequenz, das Startcodon und die ersten zwei Codons des Strukturgens der menschlichen cytoplasmatischen Cu/Zn-Superoxiddismutase durch die Sequenz XnXnXnXnATGGCXACX bereitgestellt werden, wobei jedes n unabhängig voneinander 1 ist, und jedes X unabhängig voneinander A, C, T oder G ist, und wobei die Nucleotidzusammensetzung des DNA-Spacers derart verändert ist, dass ein Expressionsniveau von mindestens 5 % w/w des gesamten zellulären Proteins erreicht wird;
(4) ein Strukturgen der menschlichen cytoplasmatischen Cu/Zn-Superoxiddismutase oder ein Allel davon im Leseraster mit dem Startcodon, das ein Stopcodon an dem 3'-Terminus des Gens aufweist; und
(5) einen Transkriptionsterminator;
wobei das DNA-Konstrukt bei Einführung in *E. coli* nichtacetylierte aktive menschliche cytoplasmatische Cu/Zn-Superoxiddismutase exprimiert.

2. Das DNA-Konstrukt gemäß Anspruch 1, wobei die Nucleotidzusammensetzung der DNA-Spacersequenz 5'-AACAATGGCAACG-3' ist.

3. Das DNA-Konstrukt gemäß Anspruch 1, wobei die Nucleotidzusammensetzung der DNA-Spacersequenz 5'-ACATATGGCAACA-3' ist.

4. Das DNA-Konstrukt gemäß Anspruch 1, wobei die Nucleotidzusammensetzung der DNA-Spacersequenz 5'-AACTATGGCAACG-3' ist.

5. Das DNA-Konstrukt gemäß Anspruch 1, wobei die Nucleotidzusammensetzung der DNA-Spacersequenz 5'-GTATATGGCTACG-3' ist.

6. Das DNA-Konstrukt gemäß Anspruch 1, wobei die Nucleotidzusammensetzung der DNA-Spacersequenz 5'-AATCATGGCAACA-3' ist.

7. Das DNA-Konstrukt gemäß Anspruch 1, wobei die Nucleotidzusammensetzung der DNA-Spacersequenz 5'-ATTTATGGCTACT-3' ist.

8. Das DNA-Konstrukt gemäß Anspruch 1, wobei das DNA-Konstrukt mit einem Replikationssystem zur extrachromosomalen Replikation und Erhaltung verbunden ist.

9. Ein Verfahren zur Herstellung eines Polypeptides, umfassend nichtacetylierte enzymatisch aktive menschliche cytoplasmatische Cu/Zn-Superoxiddismutase, die sich um nicht mehr als vier Aminosäuren von natürlich vorkommender menschlicher cytoplasmatischer Cu/Zn-Superoxiddismutase unterscheidet, wobei das Verfahren umfasst:
Kultivieren von *E. coli,* enthaltend ein DNA-Konstrukt, das einen funktionellen Vektor zur Expression in *E. coli* umfasst, in einem Nähnnedium, wobei dieses DNA-Konstrukt in abwärtslaufender Reihenfolge der Transkription umfasst:
(1) einen Promotor;
(2) eine ribosomale Bindungsstelle;
(3) ein Startcodon, wobei eine DNA-Spacersequenz zwischen der ribosomalen Bindungsstelle und dem Startcodon angeordnet ist, wobei die DNA-Spacersequenz, das Startcodon und die ersten zwei Codons des Strukturgens der menschlichen cytoplasmatischen Cu/Zn-Superoxiddismutase durch die Sequenz XnXnXnXnATGGCXACX bereitgestellt werden, wobei jedes n unabhängig voneinander 1 ist, und jedes X unabhängig voneinander A, C, T oder G ist, und wobei die Nucleotidzusammensetzung des DNA-Spacers derart verändert ist, dass ein Expressionsniveau von mindestens 5 % w/w des gesamten zellulären Proteins erreicht wird;
(4) ein Strukturgen der menschlichen cytoplasmatischen Cu/Zn-Superoxiddismutase oder ein Allel davon im Leseraster mit dem Startcodon, das ein Stopcodon an dem 3'-Terminus des Gens aufweist; und
(5) einen Transkriptionsterminator;
wobei das DNA-Konstrukt bei der Expression nichtacetylierte aktive menschliche cytoplasmatische Cu/Zn-Superoxiddismutase exprimiert; und
Isolieren der resultierenden nichtacetylierten enzymatisch aktiven menschlichen cytoplasmatischen Superoxiddismutase, welche durch die *E. coli* exprimiert wird.

## Revendications

1. Construction d'ADN comprenant un vecteur fonctionnel pour l'expression dans *E. coli*, ledit vecteur d'expression comprenant dans l'ordre de transcription vers l'aval :
(1) un promoteur
(2) un site de liaison au ribosome ;
(3) un codon d'initiation, dans lequel une séquence espaceur d'ADN est placée entre le site de liaison au ribosome et le codon d'initiation, ladite séquence espaceur d'ADN, ledit codon d'initiation et les deux premiers codons dudit gène structural de superoxyde dismutase Cu/Zn cytoplasmique humaine étant fournis par la séquence : XₙXₙXₙXₙATGGCXACX dans laquelle chaque n vaut indépendamment 1, et chaque X représente indépendamment A, C, T ou G, et dans laquelle on fait varier ladite composition nucléotidique dudit espaceur d'ADN pour obtenir un niveau d'expression d'au moins 5 % p/p de protéines cellulaires totales ;
(4) un gène structural de superoxyde dismutase Cu/Zn cytoplasmique humaine, ou l'un de ses allèles, dans le cadre de lecture avec ledit codon d'initiation, ayant un codon stop à l'extrémité 3' dudit gène ; et
(5) un terminateur de transcription,
ladite construction d'ADN, après introduction dans *E. coli,* exprimant une superoxyde dismutase Cu/Zn cytoplasmique humaine active non acétylée.

2. Construction d'ADN selon la revendication 1, dans laquelle ladite composition nucléotidique de ladite séquence espaceur d'ADN est 5'-AACAATGGCAACG-3'.

3. Construction d'ADN selon la revendication 1, dans laquelle ladite composition nucléotidique de ladite séquence espaceur d'ADN est 5'-ACATATGGCAACA-3'.

4. Construction d'ADN selon la revendication 1, dans laquelle ladite composition nucléotidique de ladite séquence espaceur d'ADN est 5'-AACTATGGCAACG-3'.

5. Construction d'ADN selon la revendication 1, dans laquelle ladite composition nucléotidique de ladite séquence espaceur d'ADN est 5'-GTATATGGCTACG-3'.

6. Construction d'ADN selon la revendication 1, dans laquelle ladite composition nucléotidique de ladite séquence espaceur d'ADN est 5'-AATCATGGCAACA-3'.

7. Construction d'ADN selon la revendication 1, dans laquelle ladite composition nucléotidique de ladite séquence espaceur d'ADN est 5'-ATTTATGGCTACT-3'.

8. Construction d'ADN selon la revendication 1, ladite construction d'ADN étant jointe à un système de réplication pour la réplication et le maintien extrachromosomiques.

9. Méthode de préparation d'un polypeptide comprenant une superoxyde dismutase Cu/Zn cytoplasmique humaine enzymatiquement active non acétylée qui ne diffère pas par plus de quatre acides aminés de la superoxyde dismutase Cu/Zn cytoplasmique humaine naturelle, ladite méthode comprenant le fait de :
faire croître *E. coli* contenant une construction d'ADN comprenant un vecteur fonctionnel pour l'expression dans *E. coli* dans un milieu nutritif, ladite construction d'ADN comprenant dans l'ordre de transcription vers l'aval :
(1) un promoteur
(2) un site de liaison au ribosome ;
(3) un codon d'initiation, dans lequel une séquence espaceur d'ADN est placée entre le site de liaison au ribosome et le codon d'initiation, ladite séquence espaceur d'ADN, ledit codon d'initiation et les deux premiers codons dudit gène structural de superoxyde dismutase Cu/Zn cytoplasmique humaine étant fournis par la séquence : XnXnXnXnATGGCXACX dans laquelle chaque n vaut indépendamment 1, et chaque X représente indépendamment A, C, T ou G, et dans laquelle on fait varier ladite composition nucléotidique dudit espaceur d'ADN pour obtenir un niveau d'expression d'au moins 5 % p/p de protéines cellulaires totales ;
(4) un gène structural de superoxyde dismutase Cu/Zn cytoplasmique humaine, ou l'un de ses allèles, dans le cadre de lecture avec ledit codon d'initiation ayant un codon stop à l'extrémité 3' dudit gène ; et
(5) un terminateur de transcription,
ladite construction d'ADN, par expression, exprimant une superoxyde dismutase Cu/Zn cytoplasmique humaine active non acétylée ; et
isoler la superoxyde dismutase résultante cytoplasmique humaine enzymatiquement active non acétylée exprimée par ladite *E. coli.*
